# EUROPÄISCHE PATENTSCHRIFT

(11) **EP 2 282 070 B1**
(45) Veröffentlichungstag und Bekanntmachung des Hinweises auf die Patenterteilung: **17.10.2012**
(21) Anmeldenummer: 09075349.2
(22) Anmeldetag: 06.08.2009
(51) Int. Cl.: F16C 1/08, A61M 1/10, A61B 17/3207, A61F 9/00, A61M 25/01

(54) **Kathetereinrichtung mit einer Ankopplungseinrichtung für eine Antriebseinrichtung**
Catheter device with a coupling device for a drive device
Dispositif de cathéter doté d'un dispositif d'accouplement pour un dispositif d'entraînement

(43) Veröffentlichungstag der Anmeldung: 09.02.2011
(73) Patentinhaber: ECP Entwicklungsgesellschaft mbH, 12247 Berlin (DE)
(72) Erfinder: Simon, Cornelia, 13187 Berlin (DE); Honselmann, Julia, 13437 Berlin (DE); Baumgärtel, Jens, 12487 Berlin (DE); Liebing, Reiner, 14469 Potsdam (DE)
(74) Vertreter: Pfenning, Meinig & Partner GbR

(56) Entgegenhaltungen:
- WO-A-01/60427
- US-A- 5 368 035
- US-A1- 2003 093 103

## Beschreibung

Die Erfindung liegt auf dem Gebiet der Mechanik bzw. des Maschinenbaus und der Feinwerktechnik und ist insbesondere beim Bau kleiner Geräte für den invasiven Betrieb in der Medizintechnik einsetzbar.

Insbesondere in der minimalinvasiven Medizin werden oft Geräte eingesetzt, die von außerhalb des Körpers über biegsame Wellen antreibbar sind. Diese Wellen werden üblicherweise durch Katheter geführt, die in den Körper durch kleine Öffnungen bzw. körpereigene Gefäße unter besonders empfindlichen Umgebungsbedingungen eingeführt werden.

Eine besondere Anwendungsform liegt beispielsweise beim Antrieb von Flüssigkeitspumpen in Mikrobauform, die z. B. als Herzpumpen eingesetzt werden und die mit einem Herzkatheter in eine Herzkammer eingeführt werden können.

Dabei liegen besondere Anforderungen nicht nur in der geringen Baugröße der entsprechenden Pumpen, wie auch beim Einsatz anderer über eine derartige Welle angetriebener mikroinvasiver Geräte, sondern auch im Betrieb der biegsamen Welle. Besonders bei den oft benötigten hohen Drehzahlen finden intensive Verformungen der Welle statt, die eine hohe mechanische und Wärmebeanspruchung hervorrufen. Daher ist es üblich, entsprechende Hohlkatheter mit einer biokompatiblen Flüssigkeit zur Schmierung und Kühlung der Welle zu füllen.

Um eine Abschottung eines derartigen Hohlkatheters gegenüber dem Körperäußeren sicherzustellen, werden häufig als Antriebe am proximalen, körperäußeren Ende der Welle Motorantriebe mit Magnetkupplungen eingesetzt, die durch hermetisch dichte Gehäusewände von entsprechenden Wellenanschlussgehäusen hindurchwirken.

Derartige Magnetkupplungen sind gegenüber herkömmlichen Kupplungen jedoch wesentlich kostenintensiver und empfindlicher. Um das erforderliche Drehmoment sicher zu übertragen, ist wegen des üblicherweise geringen Wirkungsgrades einer solchen Magnetkupplung eine erhebliche Überdimensionierung notwendig.

Zudem ist üblicherweise der Motorantrieb zusammen mit dem Hohlkatheter und der Welle und gegebenenfalls einem angeschlossenen Gerät zu zertifizieren. Dies ist insbesondere dann ineffizient, wenn wenigstens eines der Teile, entweder der Motorantrieb oder der Katheter, mehrfach verwendet werden soll und wenn diese erst beim tatsächlichen Einsatz zusammengestellt werden.

US 5 368 035 A, US 2003/0093103 A1 und WO 01/60427 A2 offenbaren heine befüllbaren Keimbarrieren.

Der vorliegenden Erfindung liegt daher die Aufgabe zugrunde, eine Kathetereinrichtung mit einem Hohlkatheter und einer beweglichen Welle zu schaffen, die in konstruktiv einfacher und kostengünstiger Weise zuverlässig und unter Einhaltung der üblichen Anforderungen an Keimfreiheit an eine entsprechende Antriebseinheit ankoppelbar ist.

Die Aufgabe wird gemäß der Erfindung mit den Merkmalen des Patentanspruchs 1 gelöst.

Dabei ist ein in einen Körper einführbarer Hohlkatheter mit einem Katheterhohlraum vorgesehen, in dem eine bewegliche Welle geführt ist, sowie eine proximale Ankopplungseinrichtung zur lösbaren Ankopplung einer Antriebseinrichtung. Die Ankopplungseinrichtung weist einen zur Antriebseinrichtung hin offenen Ankopplungshohlraum auf, in den die Welle oder ein Fortsatz der Welle mit einem Anschlusselement zur mechanischen Ankopplung einer Motorwelle hineinragt. Der Ankopplungshohlraum weist dabei eine Keimbarriere zur Verringerung der Pathogenität von krankheitserregenden Stoffen oder Mikroorganismen auf.

Auf mögliche Arten und Funktionsweisen von Keimbarrieren wird weiter unten genauer eingegangen.

Wichtig ist dabei, dass der Ankopplungshohlraum an sich zur Motorseite hin offen ist, so dass die Motorwelle in leichter und einfacher Form an die anzutreibende Welle oder einen entsprechenden Fortsatz oder ein Anschlussstück angekoppelt werden kann. Das entsprechende Anschlussstück kann dabei zudem eine Längenausgleichsmöglichkeit bereitstellen, beispielsweise durch formschlüssige Anpassung an die Motorwelle in Drehrichtung und axiale Verschiebbarkeit des Anschlussstücks gegenüber der Motorwelle. Der Ankopplungshohlraum kann beim Anschluss einer Antriebsrichtung vorteilhaft durch entsprechende Elemente der Antriebseinrichtung geschlossen werden, beispielsweise eine Hülse, die sich formschlüssig und dichtend um eine den Ankopplungshohlraum bildende Ankopplungshülse herum oder in diese hineinlegt. Wenn die Ankopplungshülse als zylindrische Hülse ausgebildet ist, kann auf Seiten der Antriebseinrichtung auch einfach ein entsprechender, vorteilhaft mit einer Dichtung versehener Deckel vorgesehen sein.

Der Ankopplungshohlraum kann durch eine Wellendichtung gegenüber dem Katheterhohlraum abgedichtet sein. Damit ergibt sich zusätzlich zu einer Keimbarriere eine Abschottung gegenüber dem Katheterhohlraum, die für sich zwar üblicherweise nicht die Anforderungen an klinische Keimfreiheit erfüllt, die jedoch einen Stoffaustausch zwischen dem Ankopplungshohlraum und dem Katheterhohlraum minimiert und damit die Anforderungen an die Keimbarriere senkt oder deren Wirkung zuverlässiger macht.

Die Ausführungsform der Keimbarriere sieht vor, dass diese als ein mit einem keimtötenden Gel füllbarer Teilraum des Ankopplungshohlraums ausgeführt ist. Dieser Teilraum ist von der Welle durchsetzt, so dass insbesondere entlang der Welle keine lebenden Keime wandern können. Die Verwendung eines Gels stellt sicher, dass dieses einerseits wegen seiner thixotropen Eigenschaften nicht fließt, d. h. ortsfest bleibt und andererseits Hohlräume, die möglicherweise entstehen könnten, immer wieder geschlossen werden. Anstelle des Gels kann gegebenenfalls auch eine zähe Flüssigkeit verwendet werden.

Es kann dabei vorgesehen sein, dass der Teilraum antriebsseitig durch eine Dichtung oder ein Lager für die Welle begrenzt ist. Zusätzlich zu der entsprechenden Stützung und Führung der Welle wird damit das Gel bzw. eventuell verflüssigte Teilmengen des Gels in dem Teilraum gehalten.

Es kann im Übrigen auch katheterhohlraumseitig eine Welle oder ein Lager vorgesehen sein, dass eine weitere Führung für die Welle darstellt, so dass der Teilraum auf beiden Seiten axial bezüglich der Welle jeweils durch ein Lager oder eine Welle begrenzt ist.

Vorteilhaft weist der Teilraum eine Füllöffnung auf, durch die bei angekoppelter Antriebseinrichtung Gel einfüllbar ist. Das heißt, dass beispielsweise in der Ankopplungshülse eine verschließbare Öffnung, z. B. mit einem Stutzen, vorgesehen ist, durch die vor oder nach Ankopplung des Antriebs das Gel einfüllbar und damit die Keimbarriere einrichtbar ist.

Eine weitere vorteilhafte Ausführungsform der Keimbarriere sieht vor, dass diese zusätzlich als ein von der Welle oder einem Fortsatz der Welle durchsetzter Bestrahlungsraum ausgebildet ist, der mit einer keimtötenden Strahlung beaufschlagbar ist. Der entsprechende Bestrahlungsraum kann als Teilraum des Ankopplungshohlraums ausgebildet sein oder diesen ganz umfassen. Es kann vorgesehen sein, dass Reflexionselemente für die Strahlung vorgesehen sind, um mit möglichst wenigen Strahlungsquellen alle Bereiche des Bestrahlungsraums erreichen zu können. Die Beschaffenheit solcher Reflexionselemente hängt von der Wellenlänge der eingesetzten Strahlung ab.

Insbesondere kann vorteilhaft der Bestrahlungsraum ein Strahlungsfenster aufweisen, das für die keimtötende Strahlung durchlässig ist und den Bestrahlungsraum hermetisch abdichtet.

Vor das Strahlungsfenster kann eine Strahlungsquelle, insbesondere eine Ultraviolettdiode, eingesetzt werden. Wenn ein entsprechendes Strahlungsfenster in die Diode integriert ist, kann auch einfach eine dichtende Aufnahme für die Diode als Strahlungsfenster dienen. Eine entsprechende Strahlungsquelle kann ggflls. auch im Bestrahlungsraum selbst angeordnet werden

Um den gesamten Bestrahlungsraum sicher mit Strahlung zu beaufschlagen, können auch zwei oder mehr Strahlungsfenster mit entsprechenden Dioden vorgesehen sein.

Die den Ankopplungshohlraum wenigstens teilweise umschließende Ankopplungshülse ist vorteilhaft mit dem Hohlkatheter hermetisch dicht, insbesondere einstückig verbunden. Die Verbindung kann durch die bekannten stoffschlüssigen Verbindungsverfahren wie Kleben und Schweißen hergestellt sein.

Ist die Kathetereinrichtung mit einem Antrieb versehen, so kann eine mit einem Antriebsgehäuse einstückig oder zumindest dicht verbundene Überwurfhülse vorgesehen sein, die auf die Ankopplungshülse außen aufschiebbar ist. Damit ist insbesondere wenn der Innendurchmesser der Überwurfhülse dem Außendurchmesser der Ankopplungshülse entspricht, ein langer Dichtungsbereich zwischen der Ankopplungshülse und der Überwurfhülse gegeben.

Die entsprechende Dichtung kann durch Einsetzen einer Elastomerdichtung zwischen der Ankopplungshülse und der Überwurfhülse noch verbessert werden.

Die Überwurfhülse weist vorteilhaft einen Schacht für die Strahlungsquelle auf, der nach dem Zusammenbau vor dem Strahlungsfenster mündet und in den die Strahlungsquelle einsetzbar ist.

Zur zuverlässigen und einfach herstellbaren Verbindung zwischen dem Hohlkatheter und der Ankopplungseinrichtung einerseits und der Antriebseinrichtung andererseits kann vorteilhaft eine rastende Verbindungseinrichtung vorgesehen sein, so dass die entsprechende Verbindung auch einfach lösbar ist. Dies ist insbesondere dann von Vorteil, wenn eines der Elemente, üblicherweise die Antriebseinrichtung, mehrfach verwendet werden soll und die Teile nach dem Einsatz bei einem Patienten getrennt werden. Die katheterseitigen Elemente werden üblicherweise in keimfreier Verpackung geliefert, nach dem Auspacken beim Patienten eingesetzt und mit einer individuell sterilisierten Antriebseinrichtung verbunden.

Um die Sicherheit des Patienten und die Funktionstüchtigkeit der Welle weiter zu erhöhen, kann vorteilhaft zudem vorgesehen sein, dass zusätzlich zu der genannten Keimbarriere auf der distalen Seite der Wellendichtung und des Ankopplungshohlraums eine zusätzliche Keimbarriere vorgesehen ist, die beispielsweise in Form einer Wellenspülvorrichtung gestaltet werden kann. Die üblicherweise eingesetzten Wellen sind, um ihre Biegsamkeit zu fördern, meistens aus dünneren Strängen zusammengesetzt und verdrillt, so dass sich eine spindelförmige Oberflächenstruktur ergibt, die bei schneller Drehung zu einer Förderung des in dem Katheter befindlichen Fluids entlang der Welle führt. Dieser Effekt ist im Allgemeinen nicht notwendigerweise erwünscht, jedoch kaum vermeidbar. Von dem antriebsseitigen Ende des Katheters her muss entsprechend neues Fluid nachströmen. Dies kann durch eine entsprechende Wellenspülvorrichtung geliefert werden, die jedoch besonderer Elemente bedarf, um das Fluid keim- und blasenfrei in den Katheterhohlraum zu fördern. Zur Ausbildung von optimierten Strömungsbedingungen innerhalb einer Wellenspülungsvorrichtung kann beispielsweise dort eine sogenannte Gegenstromhülse vorgesehen sein, die mit der Welle rotiert und Antriebselemente für das Fluid, beispielsweise in Form von Schaufelblättern, aufweist, die entgegen der Förderrichtung wirken, die durch die Wellenoberflächenstruktur auf das Fluid wirkt.

Im Zusammenwirken der Keimbarriere mit dieser zusätzlichen Keimbarriere entsteht eine zusätzliche Sicherheit für den Patienten.

Im Folgenden wird die Erfindung anhand eines Ausführungsbeispiels in einer Zeichnung gezeigt und anschließend beschrieben. Dabei zeigt
- Fig. 1: in einem schematischen Längsschnitt den Endbereich eines Hohlkatheters auf der An- triebs- oder proximalen Seite mit einem angeschlossenen Antrieb und einer Keim- barriere in Form eines gelgefüllten Teil- raums,
- Fig. 2: ebenso schematisch im Längsschnitt eine ähnliche Einrichtung wie die Fig. 1, wobei die Keimbarriere zusätzlich als Bestrahlungsraum aus- gebildet ist, sowie
- Fig. 3: schematisch die Ausbildung einer zusätz- lichen Keimbarriere in Form einer Wellen- spülvorrichtung im Endbereich des Hohl- katheters.

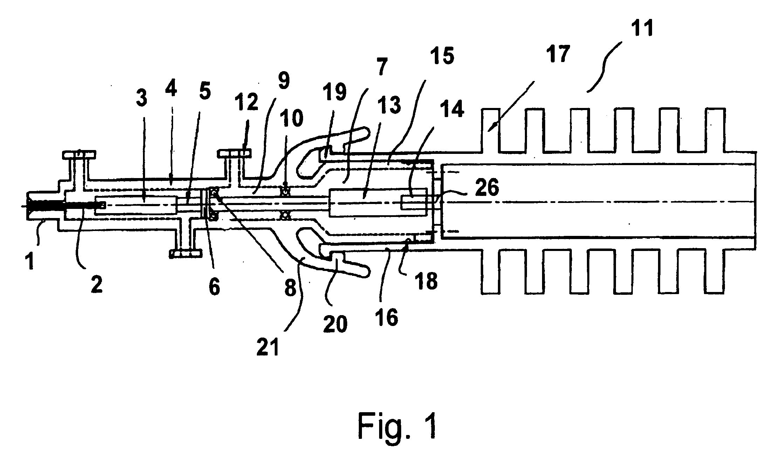

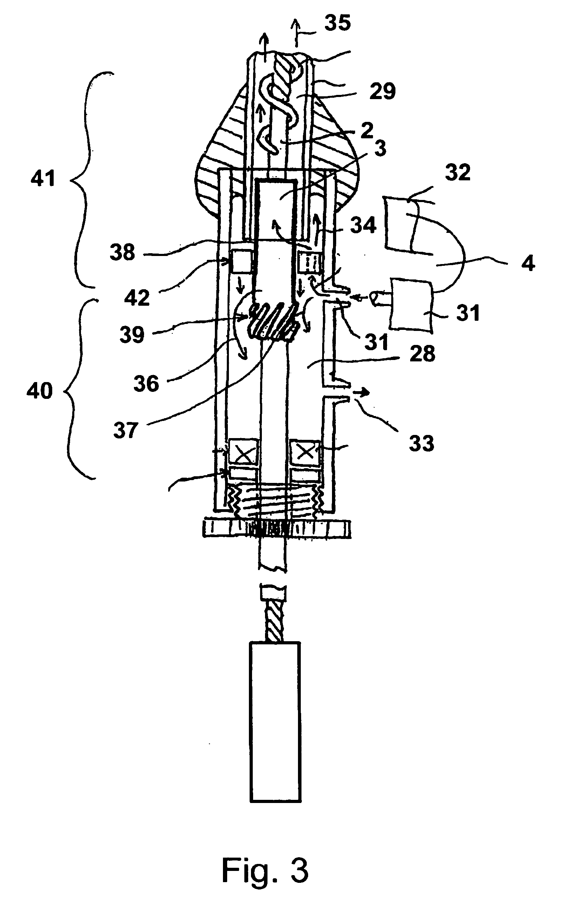

Fig. 1 zeigt auf der linken Seite den Hohlkatheter 1 mit der biegsamen Welle 2, die im Katheterhohlraum geführt und an ein erstes Anschlussstück 3 drehfest angeschlossen ist. Das erste Anschlussstück 3 liegt im Bereich einer Wellenspülvorrichtung 4, auf die weiter unten im Zusammenhang mit der Fig. 3 noch näher eingegangen wird. Jenseits des ersten Anschlussstücks 3 ist ein Fortsatz 5 der Welle 2 angeschlossen und durch eine Wellendichtung 6 dichtend von der Wellenspülvorrichtung 4 zu einem Ankopplungshohlraum 7 durchgeführt.

Jenseits der Wellendichtung 6 schließt sich ein erstes Lager 8 an, das den Wellenfortsatz 5 führt und katheterseitig einen mit Gel füllbaren oder gefüllten Teilraum 9 begrenzt. Ein zweites Lager 10 begrenzt den Teilraum 9 zur Antriebseinrichtung 11 hin.

Es ist eine Füllöffnung 12 vorgesehen, durch die der Teilraum 9 mit einem keimtötenden Gel oder einer zähflüssigen keimtötenden Flüssigkeit gefüllt werden kann. Der Wellenfortsatz 5 erstreckt sich bis zu einem zweiten Anschlussstück 13 und ist mit diesem fest verbunden.

Das zweite Anschlussstück 13 weist innen eine Vielkantöffnung, beispielsweise eine Sechskantöffnung, auf, in die ein entsprechender Sechskant 14 einer Motorwelle drehfest, jedoch zum Längenausgleich axial verschiebbar einführbar ist. Der Ankopplungshohlraum 7 wird insgesamt durch eine Ankopplungshülse 15 umschlossen, die in eine entsprechende Überwurfhülse 16 des Antriebsgehäuses 17 einführbar und an dieser mittels einer Elastomerdichtung 18 dichtbar ist. Die Überwurfhülse 16 weist Rastnasen 19 auf, die hinter entsprechende Schulterstücke 20 von Rastarmen 21 der Ankopplungshülse 15 greifen und eine lösbare Verbindung zwischen dem Antrieb 11 und dem Katheter herstellen.

Die dargestellte Einrichtung hat die Wirkung, dass der beim Montieren der Antriebseinrichtung 11 antriebsseitig offene Ankopplungshohlraum 7 von der Wellenspüleinrichtung und dem Katheterhohlraum durch eine Gelbarriere im Teilraum 9 hermetisch getrennt ist, wobei der Wellenfortsatz 5 in das Gel eingebettet ist, so dass entlang des Wellenfortsatzes 5 keine Keime vom offenen Ende des Ankopplungshohlraums zum Katheterhohlraum wandern können. Es kann somit eine Keimfreiheit auch bei Ankopplung der Antriebseinrichtung 11 in einem nicht vollständig keimfreien Raum gewährleistet werden.

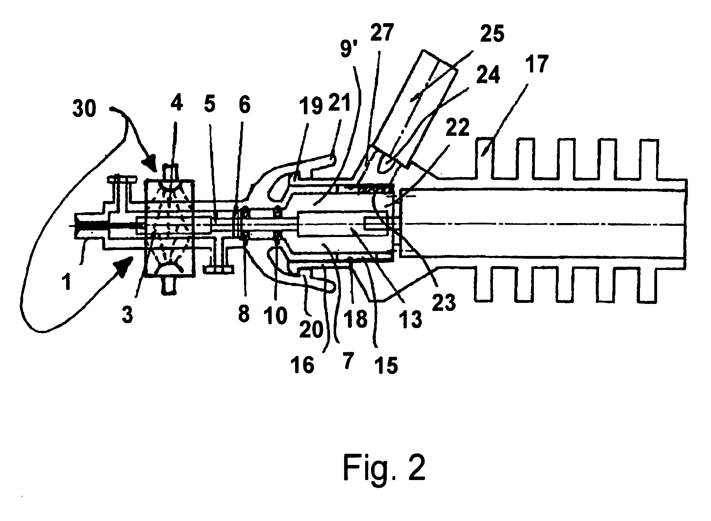

In Fig. 2 sind Elemente, die gegenüber der Fig. 1 gleich bleiben oder gleiche Funktionen ausüben, mit denselben Bezugszeichen versehen. In der Fig. 2 ist im Unterschied zur Fig. 1 als Teilraum des Ankopplungshohlraums 7, der sich von der Wellendichtung 6 bis zur antriebsseitigen Öffnung 22 des Ankopplungshohlraums erstreckt, zusätzlich ein Bestrahlungsraum 9' vorgesehen. Dieser ist unter anderem durch ein Strahlungsfenster 23 gekennzeichnet, das beispielsweise aus Plexiglas oder einem anderen für die entsprechende Strahlung, insbesondere ultraviolette Strahlung, durchlässigen Material besteht. Das Strahlungsfenster 23 ist hermetisch dicht in die Ankopplungshülse 15 eingepasst.

Antriebsseitig ist in die Überwurfhülse 16 ein Schacht 27 integriert, in den eine Strahlungsquelle in Form einer UV-Diode 24 eingesetzt werden kann, die in einer Ansteuereinheit 25 gefasst und an diese angeschlossen ist.

Es können zudem in dem Teilraum 9' Reflexionselemente für die UV-Strahlung vorgesehen sein, die im Einzelnen nicht dargestellt sind.

Der Bestrahlungsraum 9' kann zusätzlich zu dem zwischen den Lagern 8, 10 wie gemäß Fig. 1 beschrieben eingefügten Gel als Keimbarriere eingefügt werden.

Es kann grundsätzlich auch zusätzlich zu den oben beschriebenen Arten von Keimbarrieren eine Strahlungsquelle für keimtötende Strahlung, vorteilhaft eine UV- Strahlungsquelle, an einer beliebigen Position entlang der Welle bzw des Hohlkatheters, auch auf der distalen Seite der letzten Lagerdichtung, vorgesehen sein. Die Bestrahlungseinrichtung kann aufgebaut sein, wie die dargestellte Keimbarriere am Ankopplungshohlraum, sie kann jedoch auch als Überwurfmanschette mit nach innen auf den Katheter gerichteter Strahlungsquelle ausgeführt sein. Sie kann beispielsweise in Höhe der Wellenspüleinrichtung vorgesehen sein, wie in Figur 2 dargestellt und mit dem Bezugszeichen 30 versehen. Die Strahlunsquellen selbst sind dort mit 31,32 bezeichnet. Die Manschette kann wenigstens teilweise innen verspiegelt sein, um die Strahlung zu reflektieren und zu verteilen. Die Energieversorgung der Strahlungsquelle (n) kann durch parallel zum Katheter verlaufende elektrische Leitungen realisiert sein. Diese Ausführung einer Keimbarriere erfordert eine teilweise strahlungsdurchlässige Ausführung des Hohlkatheters. Sie ist grundsätzlich sowohl bei Kathetern mit einer Antriebswelle als auch ohne Antriebswelle anwendbar, zum Beispiel auch bei ein Fluid führenden und in einen Körper eingeführten Schläuchen.

Im Einzelnen in der Zeichnung nicht dargestellt ist der in dem Antriebsgehäuse 17 untergebrachte Motor, der eine in dem Sechskant 14 endende Motorwelle 26 aufweist, die elektrisch angetrieben wird.

Weiterhin am distalen Ende nicht dargestellt ist ein mit der Welle 2 verbundenes und mittels dieser antreibbares Gerät wie beispielsweise eine Mikropumpe, die als Herzpumpe einsetzbar ist, oder eine Mikrofräse.

In der Fig. 3 ist die Wellenspülvorrichtung, die sich katheterseitig an die Keimbarriere anschließt, detaillierter dargestellt. Die Keimbarriere selbst ist in der Fig. 3 der Übersichtlichkeit halber weggelassen.

Die Wellenspülvorrichtung 4 weist das erste Anschlussstück 3 auf, mit dem die Welle 2 drehfest verbunden ist. Es ist ein Spülraum 28 vorgesehen, der mit dem Katheterhohlraum 29 verbunden ist. In den Spülraum 28 kann über eine Einfüllöffnung 30 mittels einer Pumpe 31, die mit einem Reservoir 32 verbunden ist, eine keimfreie bzw. keimtötende Spülflüssigkeit eingeführt werden. Die Flüssigkeit wird teilweise durch eine Auslauföffnung 33, die auch der Entlüftung dient, wieder abgeführt.

Die Spülflüssigkeit verteilt sich gemäß den dargestellten Flussrichtungspfeilen 34, 35 in Richtung des Katheterhohlraums und an der Welle 2 entlang sowie von der Einfüllöffnung 30 aus in der entgegengesetzten Richtung, wie durch die Pfeile 36, 37 dargestellt, zur Antriebsseite hin. Dies wird insbesondere durch die Gegenstromhülse 38 bewirkt, die Antriebselemente 39 in Form von schaufelblattartigen Stegen aufweist und die sich mit dem ersten Anschlussstück 3 dreht. Die Gegenstromhülse 38 kann auch einstückig mit dem Anschlussstück 3 zusammenhängen.

Durch die Gegenstromhülse wird im axialen Bereich, der mit 40 bezeichnet ist, innerhalb des Spülraums 28 ein leichter Überdruck erzeugt, der das Eindringen von Luft durch die Entlüftungsöffnung 33 verhindert oder wenigstens vermindert. Gleichzeitig wird in dem mit 41 bezeichneten axialen Raum und im gesamten Katheterhohlraum 39 der Druck etwas abgesenkt und damit der Fluss des Spülmittels in Richtung der Welle zum distalen Ende hin verhindert. Dies ist wünschenswert, um den Stoffdurchsatz entlang der Welle zu reduzieren.

Zur Lagerung der Gegenstromhülse 38 ist ein für die Spülflüssigkeit durchlässiges Lager 42 vorgesehen.

Insbesondere zusammen mit den in Fign. 1 und 2 detaillierter dargestellten Keimbarrieren verbessert die Spülvorrichtung zusätzlich die Keimfreiheit des Katheters und gewährleistet einen verschleißarmen und zuverlässigen Betrieb der Welle auch bei hohen Drehzahlen.

Die Anmeldungsgegenstand umfasst unter anderem die folgenden Aspekte:
1. Kathetereinrichtung mit einem Hohlkatheter 1, in dessen Katheterhohlraum 29 eine bewegliche Welle 2 geführt ist, mit einer proximalen Ankopplungseinrichtung zur lösbaren Ankopplung einer Antriebseinrichtung, wobei die Ankopplungseinrichtung einen zur Antriebseinrichtung hin offenen Ankopplungshohlraum aufweist, in den die Welle oder ein Fortsatz 5 der Welle 82 mit einem Anschlusselement 13 zur mechanischen Ankopplung einer Motorwelle 26 hineinragt, wobei der Ankopplungshohlraum 7 eine Keimbarriere 9, 9' zur Verringerung der Pathogenität von krankheitserregenden Stoffen oder Mikroorganismen aufweist.
2. Kathetereinrichtung nach Aspekt 1, dadurch gekennzeichnet, dass der Ankopplungshohlraum 7 durch eine Wellendichtung 6 gegenüber dem Katheterhohlraum abgedichtet ist.
3. Kathetereinrichtung nach Aspekt 1 oder 2, dadurch gekennzeichnet, dass eine Keimbarriere als ein mit einem keimtötenden Gel füllbarer Teilraum 9 ausgeführt ist.
4. Kathetereinrichtung nach Aspekt 3, dadurch gekennzeichnet, dass der Teilraum 9 antriebsseitig durch eine Dichtung oder ein Lager 10 für die Welle 2, 5 begrenzt ist.
5. Kathetereinrichtung nach Aspekt 3 oder 4, dadurch gekennzeichnet, dass der Teilraum eine Füllöffnung 12 aufweist, durch die bei angekoppelter Antriebseinrichtung 11 ein Gel einfüllbar ist.
6. Kathetereinrichtung nach Aspekt 3, 4 oder 5, dadurch gekennzeichnet, dass der abschottbare Teilraum antriebsseitig und katheterhohlraumseitig durch eine Dichtung oder ein Lager 8, 10 begrenzt ist.
7. Kathetereinrichtung nach Aspekt 1 oder einem der folgenden, dadurch gekennzeichnet, dass eine Keimbarriere als ein von der Welle 2 oder einem Fortsatz 5 der Welle durchsetzter Bestrahlungsraum 9' ausgebildet ist, der mit einer keimtötenden Strahlung beaufschlagbar ist.
8. Kathetereinrichtung nach Aspekt 7, dadurch gekennzeichnet, dass der Bestrahlungsraum ein Strahlungsfenster 23 aufweist, das den Bestrahlungsraum 9' hermetisch abdichtet.
9. Kathetereinrichtung nach Aspekt 8, dadurch gekennzeichnet, dass vor dem Strahlungsfenster eine Strahlungsquelle, insbesondere eine UV-Diode 24 einsetzbar ist.
10. Kathetereinrichtung nach Aspekt 1 oder einem der folgenden, dadurch gekennzeichnet, dass eine den Ankopplungshohlraum 7 wenigstens teilweise umschließende Ankopplungshülse mit dem Hohlkatheter hermetisch dicht, insbesondere einstückig verbunden ist.
11. Kathetereinrichtung nach Aspekt 8 oder 9 mit einer Antriebseinrichtung, dadurch gekennzeichnet, dass ein mit einem Antriebsgehäuse verbundener Schacht 27 bei angekoppelter Antriebseinrichtung an dem Bestrahlungsfenster mündet und eine UV-Diode 24 aufnimmt.
12. Kathetereinrichtung nach Aspekt 10 mit einer Antriebseinrichtung, dadurch gekennzeichnet, dass eine mit einem Antriebsgehäuse 17 einstückig verbundene oder verklebte Überwurfhülse 16 vorgesehen ist, die auf die Ankopplungshülse 15 außen aufschiebbar ist.
13. Kathetereinrichtung mit einem Antrieb nach Aspekt 12, dadurch gekennzeichnet, dass eine Dichtung, insbesondere eine Elastomerdichtung 18 zwischen der Ankopplungshülse 15 und der Überwurfhülse 16 vorgesehen ist.
14. Kathetereinrichtung nach Aspekt 12 oder 13, dadurch gekennzeichnet, dass in der Überwurfhülse der Schacht 27 für die Strahlungsquelle 24 vorgesehen ist.
15. Kathetereinrichtung nach Aspekt 10, 11, 12, 13 oder 14, dadurch gekennzeichnet, dass eine rastende Verbindungseinrichtung 19, 20 zur Herstellung einer lösbaren Verbindung zwischen der Ankopplungshülse 15 und einem Antriebsgehäuse 17 vorgesehen ist.
16. Kathetereinrichtung nach Aspekt 2 oder einem der folgenden, dadurch gekennzeichnet, dass auf der distalen Seite der Wellendichtung 6 eine zusätzliche Keimbarriere vorgesehen ist, insbesondere in Form einer Wellenspülvorrichtung 4.

## Patentansprüche

1. Kathetereinrichtung mit einem Hohlkatheter (1), in dessen Katheterhohlraum (29) eine bewegliche Welle (2) geführt ist, mit einer proximalen Ankopplungseinrichtung zur lösbaren Ankopplung einer Antriebseinrichtung, wobei die Ankopplungseinrichtung einen zur Antriebseinrichtung hin offenen Ankopplungshohlraum aufweist, in den die Welle oder ein Fortsatz (5) der Welle (82) mit einem Anschlusselement (13) zur mechanischen Ankopplung einer Motorwelle (26) hineinragt, wobei der Ankopplungshohlraum (7) eine Keimbarriere (9,9') zur Verringerung der Pathogenität von krankheitserregenden Stoffen oder Mikroorganismen aufweist, **dadurch gekennzeichnet, dass** eine Keimbarriere als ein mit einem keimtötenden Gel füllbarer Teilraum (9) ausgeführt ist.

2. Kathetereinrichtung nach Anspruch 1, **dadurch gekennzeichnet, dass** der Ankopplungshohlraum (7) durch eine Wellendichtung (6) gegenüber dem Katheterhohlraum abgedichtet ist.

3. Kathetereinrichtung nach Anspruch 1, **dadurch gekennzeichnet, dass** der Teilraum (9) antriebsseitig durch eine Dichtung oder ein Lager (10) für die Welle (2,5) begrenzt ist.

4. Kathetereinrichtung nach Anspruch 2 oder 3, **dadurch gekennzeichnet, dass** der Teilraum eine Füllöffnung (12) aufweist, durch die bei angekoppelter Antriebseinrichtung (11) ein Gel einfüllbar ist.

5. Kathetereinrichtung nach Anspruch 3 oder 4, **dadurch gekennzeichnet, dass** der abschottbare Teilraum antriebsseitig und katheterhohlraumseitig durch eine Dichtung oder ein Lager (8,10) begrenzt ist.

6. Kathetereinrichtung nach Anspruch 1 oder einem der folgenden, **dadurch gekennzeichnet, dass** eine Keimbarriere als ein von der Welle (2) oder einem Fortsatz (5) der Welle durchsetzter Bestrahlungsraum (9') ausgebildet ist, der mit einer keimtötenden Strahlung beaufschlagbar ist.

7. Kathetereinrichtung nach Anspruch 6, **dadurch gekennzeichnet, dass** der Bestrahlungsraum ein Strahlungsfenster(23) aufweist, das den Bestrahlungsraum (9') hermetisch abdichtet.

8. Kathetereinrichtung nach Anspruch 7, **dadurch gekennzeichnet, dass** vor dem Strahlungsfenster eine Strahlungsquelle, insbesondere eine UV-Diode (24) einsetzbar ist.

9. Kathetereinrichtung nach Anspruch 1 oder einem der folgenden, **dadurch gekennzeichnet, dass** eine den Ankopplungshohlraum (7) wenigstens teilweise umschließende Ankopplungshülse mit dem Hohlkatheter hermetisch dicht, insbesondere einstückig verbunden ist.

10. Kathetereinrichtung nach Anspruch 7 oder 8 mit einer Antriebseinrichtung, **dadurch gekennzeichnet, dass** ein mit einem Antriebsgehäuse verbundener Schacht (27) bei angekoppelter Antriebseinrichtung an dem Bestrahlungsfenster mündet und eine UV-Diode (24) aufnimmt.

11. Kathetereinrichtung nach Anspruch 9 mit einer Antriebseinrichtung, **dadurch gekennzeichnet, dass** eine mit einem Antriebsgehäuse (17) einstückig verbundene oder verklebte Überwurfhülse (16) vorgesehen ist, die auf die Ankopplungshülse (15) außen aufschiebbar ist.

12. Kathetereinrichtung mit einem Antrieb nach Anspruch 11, **dadurch gekennzeichnet, dass** eine Dichtung, insbesondere eine Elastomerdichtung (18) zwischen der Ankopplungshülse (15) und der Überwurfhülse (16) vorgesehen ist.

13. Kathetereinrichtung nach Anspruch 11 oder 12, **dadurch gekennzeichnet, dass** in der Überwurfhülse der Schacht (27) für die Strahlungsquelle(24)vorgesehen ist.

14. Kathetereinrichtung nach Anspruch 9, 10, 11, 12 oder 13, **dadurch gekennzeichnet, dass** eine rastende Verbindungseinrichtung (19,20) zur Herstellung einer lösbaren Verbindung zwischen der Ankopplungshülse (15) und einem Antriebsgehäuse (17) vorgesehen ist.

15. Kathetereinrichtung nach Anspruch 2 oder einem der folgenden, **dadurch gekennzeichnet, dass** auf der distalen Seite der Wellendichtung (6) eine zusätzliche Keimbarriere vorgesehen ist, insbesondere in Form einer Wellenspülvorrichtung (4).

## Claims

1. Catheter device having a hollow catheter (1) in the catheter cavity (29) of which a moveable shaft (2) is guided, having a proximal coupling device for releasable coupling of a drive device, the coupling device having a coupling cavity which is open towards the drive device and into which the shaft or an extension (5) of the shaft (2) having a connection element (13) for mechanical coupling of a motor shaft (26) protrudes, the coupling cavity (7) having a germ barrier (9, 9') for reducing the pathogenicity of pathogenic substances or microorganisms, **characterised in that** a germ barrier is configured as a partial chamber (9) which can be filled with a germicidal gel.

2. Catheter device according to claim 1, **characterised in that** the coupling cavity (7) is sealed by a shaft seal (6) relative to the catheter cavity.

3. Catheter device according to claim 1, **characterised in that** the partial chamber (9) is delimited, on the drive side, by a seal or a bearing (10) for the shaft (2, 5).

4. Catheter device according to claim 2 or 3, **characterised in that** the partial chamber has a filling opening (12) through which a gel can be introduced when the drive device (11) is coupled.

5. Catheter device according to claim 3 or 4, **characterised in that** the shieldable partial chamber is delimited, on the drive side and on the catheter cavity side, by a seal or a bearing (8, 10).

6. Catheter device according to claim 1 or one of the following, **characterised in that** a germ barrier is configured as an irradiation chamber (9') which is penetrated by the shaft (2) or an extension (5) of the shaft and can be supplied with a germicidal radiation.

7. Catheter device according to claim 6, **characterised in that** the irradiation chamber has a radiation window (23) which hermetically seals the irradiation chamber (9').

8. Catheter device according to claim 7, **characterised in that** a radiation source, in particular a UV diode (24), can be inserted in front of the radiation window.

9. Catheter device according to claim 1 or one of the following, **characterised in that** a coupling sleeve which surrounds the coupling cavity (7) at least partially is connected in a hermetically sealed manner, in particular in one piece, to the hollow catheter.

10. Catheter device according to claim 7 or 8 having a drive device, **characterised in that** a shaft (27) connected to a drive housing opens at the irradiation window, when the drive device is coupled, and receives a UV diode (24).

11. Catheter device according to claim 9 having a drive device, **characterised in that** a cover sleeve (16) which is connected or glued in one piece to a drive housing (17) is provided and can be pushed onto the coupling sleeve (15) from outside.

12. Catheter device having a drive according to claim 11, **characterised in that** a seal, in particular an elastomer seal (18), between the coupling sleeve (15) and the cover sleeve (16) is provided.

13. Catheter device according to claim 11 or 12, **characterised in that** the shaft (27) for the radiation source (24) is provided in the cover sleeve.

14. Catheter device according to claim 9, 10, 11, 12 or 13, **characterised in that** a locking connection device (19, 20) for producing a detachable connection between the coupling sleeve (15) and a drive housing (17) is provided.

15. Catheter device according to claim 2 or one of the following, **characterised in that** an additional germ barrier, in particular in the form of a shaft rinsing device (4), is provided on the distal side of the shaft seal (6).

## Revendications

1. Dispositif de cathéter avec un cathéter creux (1), dans la cavité de cathéter (29) duquel on insère un arbre mobile (2), avec un dispositif de couplage proximal servant au couplage amovible d'un dispositif d'entraînement, dans lequel le dispositif de couplage présente une cavité de couplage ouverte en direction du dispositif d'entraînement, cavité de couplage dans laquelle pénètre l'arbre ou un prolongement (5) de l'arbre (82) avec un élément de raccordement (13) pour le couplage mécanique d'un arbre moteur (26), dans lequel la cavité de couplage (7) présente une barrière contre les germes (9, 9') pour une réduction de la pathogénie de substances ou de microorganismes pathogènes, **caractérisé en ce qu'**une barrière contre les germes est réalisée sous la forme d'un sous-espace (9) pouvant être rempli avec un gel germicide.

2. Dispositif de cathéter selon la revendication 1, **caractérisé en ce que** la cavité de couplage (7) est étanchéifiée vis-à-vis de la cavité de cathéter par un joint d'étanchéité d'arbre (6).

3. Dispositif de cathéter selon la revendication 1, **caractérisé en ce que** le sous-espace (9) est délimité, côté entraînement, par un joint ou un palier (10) pour l'arbre (2, 5).

4. Dispositif de cathéter selon la revendication 2 ou 3, **caractérisé en ce que** le sous-espace présente un orifice de remplissage (12) à travers lequel on peut verser un gel lorsque le dispositif d'entraînement (11) est raccordé.

5. Dispositif de cathéter selon la revendication 3 ou 4, **caractérisé en ce que** le sous-espace cloisonnable est délimité, côté entraînement et côté cavité de cathéter, par un joint ou un palier (8, 10).

6. Dispositif de cathéter selon la revendication 1 ou l'une quelconque des revendications suivantes, **caractérisé en ce qu'**une barrière contre les germes se présente sous la forme d'un espace d'irradiation (9') traversé par l'arbre (2) ou un prolongement (5) de l'arbre, lequel espace d'irradiation est exposable à une radiation germicide.

7. Dispositif de cathéter selon la revendication 6, **caractérisé en ce que** l'espace d'irradiation présente une fenêtre de radiation (23) qui étanchéifie hermétiquement l'espace d'irradiation (9').

8. Dispositif de cathéter selon la revendication 7, **caractérisé en ce qu'**une source de radiation, en particulier une diode UV (24), peut être installée devant la fenêtre de radiation.

9. Dispositif de cathéter selon la revendication 1 ou l'une quelconque des revendications suivantes, **caractérisé en ce qu'**une douille de couplage, entourant au moins partiellement la cavité de couplage (7), est reliée au cathéter creux de manière hermétiquement étanchéifiée, en particulier en un seul tenant.

10. Dispositif de cathéter selon la revendication 7 ou 8 avec un dispositif d'entraînement, **caractérisé en ce qu'**un puits (27), relié à un boîtier d'entraînement, débouche sur la fenêtre d'irradiation lorsque le dispositif d'entraînement est couplé et héberge une diode UV (24).

11. Dispositif de cathéter selon la revendication 9 avec un dispositif d'entraînement, **caractérisé en ce qu'**il est prévu une douille de recouvrement (16) reliée d'un seul tenant à un boîtier d'entraînement (17) ou collée à celui-ci, laquelle douille de recouvrement peut être coulissée à l'extérieur sur la douille de couplage (15).

12. Dispositif de cathéter avec un entraînement selon la revendication 11, **caractérisé en ce qu'**il est prévu un joint, en particulier un joint élastomère (18), entre la douille de couplage (15) et la douille de recouvrement (16).

13. Dispositif de cathéter selon la revendication 11 ou 12, **caractérisé en ce que** le puits (27) pour la source de radiation (24) est prévu dans la douille de recouvrement.

14. Dispositif de cathéter selon les revendications 9, 10, 11, 12 ou 13, **caractérisé en ce qu'**il est prévu un dispositif de raccordement encliquetable (19, 20) pour l'établissement d'une liaison amovible entre la douille de couplage (15) et un boîtier d'entraînement (17).

15. Dispositif de cathéter selon la revendication 2 ou l'une quelconque des revendications suivantes, **caractérisé en ce que**, sur le côté distal du joint d'étanchéité d'arbre (6), est prévue une barrière supplémentaire contre les germes, en particulier sous la forme d'un dispositif de rinçage de l'arbre (4).
